# EUROPEAN PATENT APPLICATION

(11) **EP 1 604 643 A1**
(43) Date of publication of application: **14.12.2005**
(21) Application number: 05011282.0
(22) Date of filing: 25.05.2005
(51) Int. Cl.: A61K 7/40, A61K 7/48, A61K 7/06

(54) **Cosmetic use of derivatives of 3-Phenylpropionic acid**

(30) Priority: 03.06.2004 EP 04291389
(71) Applicant: Cognis France, S.A.S., 31360 Boussens (FR); Cognis IP Management GmbH, 40589 Düsseldorf (DE)
(72) Inventor: Speitkamp, Marc, 40476 Düsseldorf (DE); Markert, Thomas, 40789 Monheim (DE); Rathjens, Andreas, 54510 Tomblaine (FR); Danoux, Louis, 54420 Saulxures les Nancy (FR); Moussou, Philippe, 54000 Nancy (FR)
(74) Representative: Gittinger, Andreas

(57) **Abstract**

The use of a compound having formula I 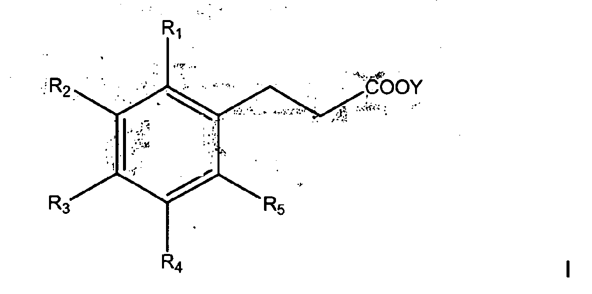 wherein Y is selected from the group consisting of H, an alkyl group, a phenyl group, Na, K or NH₄, and wherein R₁, R₂, R₃, R₄ and R₅ are independently selected from the group consisting of H and -O-R₆, wherein R₆ is selected from the group consisting of H and a linear or branched, saturated or unsaturated alkyl group comprising 1 to 6 carbon atoms, and wherein at least one of the substituents R₁, R₂, R₃, R₄ and R₅ is not H, for the manufacture of a composition for the cosmetic treatment of human skin, scalp or hair and for the cosmetic treatment of human skin, scalp or hair.

## Description

The use of a compound having formula I 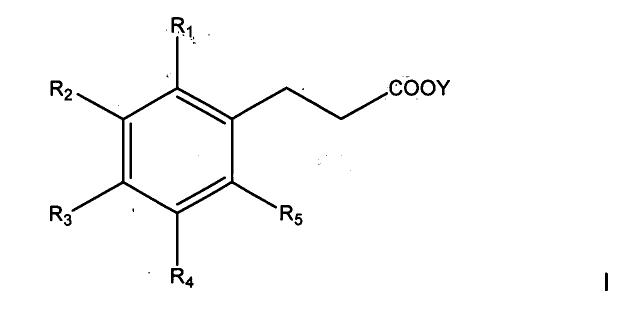 wherein Y is selected from the group consisting of H, an alkyl group, a phenyl group, Na, K or NH₄, and wherein R₁, R₂, R₃, R₄ and R₅ are independently selected from the group consisting of H and -O-R₆, wherein R₆ is selected from the group consisting of H and a linear or branched, saturated or unsaturated alkyl group comprising 1 to 6 carbon atoms, and wherein at least one of the substituents R₁, R₂, R₃, R₄ and R₅ is not H, for the manufacture of a composition for the cosmetic treatment of human skin, scalp or hair and for the cosmetic treatment of human skin, scalp or hair.

Some alkoxy substituted phenylpropanoic acids, e. g. dihydrocaffeic acid, dihydroferulic acid, dihydroumbellic acid or dihydrosinapinic acid are known for their antioxydant activities.

Dihydrosinapinic acid has the following formula

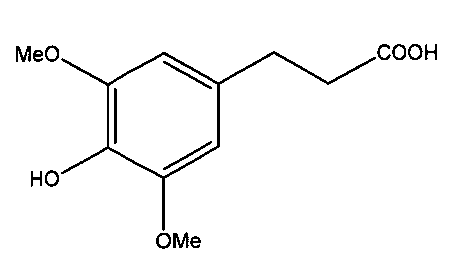

The chemical name of dihydrosinapinic acid is 3-(4-hydroxy-3,5-dimethoxyphenyl) propionic acid or 4-hydroxy-3,5-dimethoxyhydrocinnamic acid or 4-hydroxy-3,5-dimethoxy-benzenepropanoic acid or 4-hydroxy-3,5-dimethoxy-hydrocinnamic acid. The isolation of dihydrosinapinic acid and dihydroferulic acid from Kurosu (unpolished rice vinegar) and their antioxydant activity in DPPH radical scavenging is has been described by Shimoji Yumi et al. (**Journal of Agricultural and Food Chemistry (2002), volume 50, pages 6501-6503).** Their use as health foods for preventing carcinogenesis, and their antitumor effect against human lung, mammary, bladder and prostate cancer cell lines were disclosed In **JP 2003-095976** (Tamanoi Vinegar Co., Ltd., Japan).

The isolation of dihydrosinapinic acid from kimchi or its chemical synthesis was described in **WO 02/074300** (Dong Wa Pharm Ind. Co., Ltd., S. Korea), together with their inhibitory effect against HMG-CoA reductase activity, and their use in composition for treating arteriosclerosis.

The manufacture of antioxydants including dihydrosinapinic acid from plants such as corn, coffee beans, grapes for controlling oxidation in food as well as in pharmaceuticals in human bodies is described in **JP 11172246** (Sanei Toka K.K., Japan).

Surprisingly it has been discovered that a compound having formula I 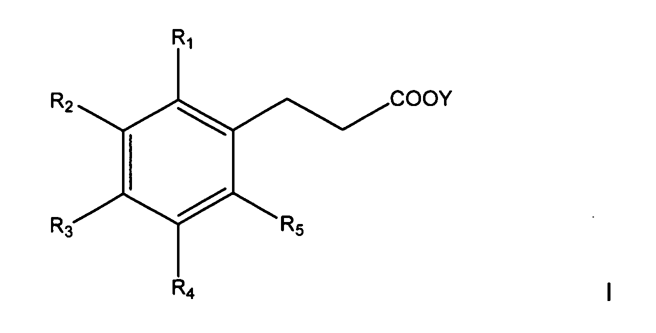 wherein Y is selected from the group consisting of H, an alkyl group, a phenyl group, Na, K or NH₄, and wherein R₁, R₂, R₃, R₄ and R₅ are independently selected from the group consisting of H and -O-R₆, wherein R₆ is selected from the group consisting of H and a linear or branched, saturated or unsaturated alkyl group comprising 1 to 6 carbon atoms, and wherein at least one of the substituents R₁, R₂, R₃, R₄ and R₅ is not H, is useful for the manufacture of a composition for the cosmetic treatment of human skin, scalp or hair and that this compound is useful for the cosmetic treatment of human skin, scalp or hair.

These two said uses are one subject of the present invention. These two uses are called the use according to the present invention.

One embodiment of the present invention is this use according to the present invention, wherein Y is selected from the group consisting of H, methyl, ethyl and propyl, and wherein R₁ = R₂ = H, and wherein R₃, R₄ and R₅ are independently selected from the group consisting of H, -OH and -O-methyl, and wherein at least one of the substituents R₃, R₄ and R₅ is not H.

Another embodiment of the present invention is this use according to the present invention, wherein the compound is dihydrosinapinic acid.

Another embodiment of the present invention is this use of the compound for the manufacture of a composition according to the present invention (or the use of the compound in a composition), wherein the composition comprises the compound having formula I and at least one cosmetic adjuvant.

Another embodiment of the present invention is this use according to the present invention, wherein the cosmetic adjuvant is selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

Another embodiment of the present invention is this use according to the present invention, wherein the cosmetic treatment comprises the bringing about of an anti-ageing or anti-photoageing effect (related to UV irradiation or IR irradiation) or a protecting effect against UV-A-radiation ("UV-A-cytophotoprotection") or an anti-inflammatory, or an anti-irritant effect, or an anti-itching effect or an appeasing effect or an anti-protease effect or an anti-lipoxygenase effect.

Particular benefits achieved by the cosmetic use of the compounds having formula I (alkoxy-substituted phenylpropanoic acids: or derivatives) are
- the protection of skin and scalp against skin ageing or photo-ageing damages such as macromolecular matrix proteins degradation
- the protection of skin and scalp against UVA induced damages, oxidative stress, environmental stress or against pollutants, or the use of the compounds having formula I as anti-inflammatory, anti-itching or appeasing agent,
- the stability in cosmetic formulations of the compounds having formula I.

The following paragraphs list preferred embodiments and advantages of the present invention as well as some background information.
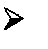 The invention concerns the cosmetic use of alkoxy-substituted phenylpropanoic acids of general formula I to protect the skin and scalp against ageing and photo-ageing (UV or/and Infra-red) damages.
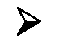 It is well known that intrinsic ageing and/or photo-ageing (due to UV or Infra-red) modify the skin particularly by provoking modification in the synthesis of skin structure proteins such as collagens, GAGs (glycosaminoglycans), proteoglycans and elastin causing a lot of damages to extracellular matrix of dermis. The alkoxy-substituted phenyl propanoic acids of general formula I may be used to counterbalance these deleterious effects.
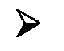 The invention furthermore concerns the cosmetic use of alkoxy-substituted phenylpropanoic acids of general formula I to protect the skin and scalp against damages caused by UVA radiation. It has been found that the alkoxy-substituted phenylpropanoic acids according to the invention protect skin fibroblasts cells against UVA radiation. The UVA radiation (320-400 nm) is a penetrating radiation which affects the dermis. The generation of reactive oxygen species like anion superoxide, hydrogen peroxide and singlet oxygen, which are responsible for lipid peroxidation, protein damages or mitochondrial DNA damages, is induced by UVA radiation, and involves immediate and transient biological responses for example inflammation, sunburn, loss of skin cellularity and elasticity, and delayed and chronic biological responses such as photoageing, or photocarcinogenesis.
The invention furthermore concerns the cosmetic use of alkoxy-substituted phenylpropanoic acids of general formula I to protect the skin and scalp against local inflammations. It has been found that the alkoxy-substituted phenylpropanoic acids according to the invention inhibit the "respiratory burst" on an activated human leukocytes cell line. During cutaneous inflammation, leukocytes are activated by peptides like cytokines and others messengers like Leukotriens released from activated or necrotic epidermal cells. This activation of leukocytes as polymorphonuclear neutrophilic granulocytes (PMN), known as "respiratory burst" provokes the release of reactive oxygen species (ROS) and lysosomal enzymes which can mediate many tissue damages.
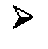 The alkoxy-substituted phenylpropanoic acids according to the present invention may be used to protect the skin and scalp and/or to fight against UV, Infra-red and sun damages, sunburn, mitochondrial DNA damages, to prevent or fight against photo-aging, to provide improvement for signs of ageing as skin wrinkles and elasticity loss.
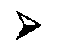 They may be used also to protect skin, scalp and/or hair shaft and fight against oxidative or stress damages, to protect skin, scalp and/or hair shaft from environmental stress such as pollutants, chemicals.
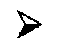 They may be used to improve the appearance of the skin with local inflammations, erythema or microinflammations. Moreover, they may be used to treat sensitive or irritated skin or scalp, as appeasing, anti-irritant and anti-itching agent.
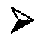 The disclosed alkoxy-substituted phenylpropanoic acids show a very good chemical stability. They do not cause any coloration or degradation of the cosmetic formulations, and are well tolerated by skin.

The following paragraph describes the synthesis of the compounds having formula I.

The compounds having formula I can be synthesized using conventional chemical methods, e. g. according to the Perkin method or according to the Knoevenagel method. The compounds having formula I can also be obtained by extracting them from plants, in which they occur naturally. The compounds having formula I can also be obtained by biotechnological methods. The compounds having formula I can also be obtained by a combination of these methods.

The dose of use of the compounds having formula I preferably ranges from 0.0001 % to 10%, more preferably from 0.001% to 5%, and more preferably from 0.01% to 2%, relative to the total weight of the composition.

The alkoxy substituted phenylpropanoic acids or derivatives according to the present invention may be used alone or in combination with other active products, such as plant extracts, proteins or protein hydrolysates, amino-acids, vitamins, polyols, polysaccharide or oligosaccharide.

Alkoxy substituted phenylpropanoic acids or derivatives according to the present invention can be encapsulated in liposomes, in beta-cyclodextrine, in a hydrophobic matrix, or adsorbed on starch. To protect the active principle from photo-oxidative damage, heavy metals catalyzed and free-radicals induced oxidation, formulation with other antioxidants, with metal-chelating compounds and/or with UV-filters may be used.

The cosmetic adjuvant of the present invention can be selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

In one embodiment of the present invention the cosmetic adjuvant is selected from the group consisting of surfactants, emulsifiers, fats, waxes, stabilizers, deodorants, antiperspirants, antidandruff agents and perfume oils.

The total content of the cosmetic adjuvant may be 1 to 50% by weight, preferably 5 to 40% by weight, based on the cosmetic preparations (cosmetic compositions). The preparations can be prepared by customary cold or hot processes; preference is given to using the phase-inversion temperature method.

For the purposes of the invention, cosmetic preparations can mean care agents. Care agents are understood as meaning care agents for skin and hair. These care agents include, inter alia, cleansing and restorative action for skin and hair.

Application can be topical or oral in the form of tablets, dragees, capsules, juices, solutions and granules.

The compositions and cosmetic preparations according to the invention can be used for the preparation of cosmetic and/or dermopharmaceutical preparations, e. g. hair shampoos, hair lotions, foam baths, shower baths, creams, gels, lotions, alcoholic and aqueous/alcoholic solutions, emulsions, wax/fat compositions, stick preparations, powders or ointments. Furthermore, the preparations for oral application according to the invention can also be incorporated into tablets, dragees, capsules, juices, solutions and granules.

Surfactants (or Surface-active substances) that may be present are anionic, non-ionic, cationic and/or amphoteric or amphoteric surfactants, the content of which in the compositions is usually about 1 to 70% by weight, preferably 5 to 50% by weight and in particular 10 to 30% by weight. Typical examples of anionic surfactants are soaps, alkylbenzenesulfonates, alkanesulfonates, olefin sulfonates, alkyl ether sulfonates, glycerol ether sulfonates, α-methyl ester sulfonates, sulfo fatty acids, alkyl sulphates, fatty alcohol ether sulphates, glycerol ether sulphates, fatty acid ether sulphates, hydroxy mixed ether sulphates, monoglyceride (ether) sulphates, fatty acid amide (ether) sulphates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids, e. g. acyl lactylates, acyl tartrates, acyl glutamates and acyl aspartates, alkyl oligoglucoside sulphates, protein fatty acid condensates (in particular wheat-based vegetable products) and alkyl (ether) phosphates. If the anionic surfactants contain polyglycol ether chains, these may have a conventional homologous distribution, but preferably have a narrowed homologous distribution. Typical examples of non-ionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers or mixed formals, optionally partially oxidized alk(en)yl oligoglycosides or glucoronic acid derivatives, fatty acid N-alkylglucamides, protein hydrolysates (in particular wheat-abased vegetable products), polyol fatty acid esters, sugar esters, sorbitan esters, polysorbates and amine oxides. If the non-ionic surfactants contain polyglycol ether chains, these may have a conventional homologous distribution, but preferably have a narrowed homologous distribution. Typical examples of cationic surfactants are quaternary ammonium compounds, e. g. dimethyldistearylammonium chloride, and ester quats, in particular quaternized fatty acid trialkanolamine ester salts. Typical examples of amphoteric or zwitterionic surfactants are alkylbetaines, alkylamidobetaines, aminopropionates, aminoglycinates, imidazolinium-betaines and sulfobetaines. Said surfactants are known compounds. With regard to structure and preparation of these substances, reference may be made to relevant review works.

Typical examples of particularly suitable mild, i.e. particularly skin-compatible surfactants are fatty alcohol polyglycol ether sulphates, monoglyceride sulphates, mono-and/or dialkyl sulfosuccinates, fatty acid isethionates, fatty acid sarcosinates, fatty acid taurides, fatty acid glutamates, α-olefinsulfonates, ether carboxylic acids, alkyl oligoglucosides, fatty acid glucamides, alkylamidobetaines, amphoacetals and/or protein fatty acid condensates, the latter preferably based on wheat proteins.

Suitable oily bodies are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, for example myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxycarboxylic acids with linear or branched C₆-C₂₂-fatty alcohols, in particular dioctyl malates, esters of linear and/or branched fatty acids with polyhydric alcohols (for example propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, for example dicaprylyl carbonates (Cetiol® CC), Guerbet carbonates based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or unsymmetrical dialkyl ethers having 6 to 22 carbon atoms per alkyl group, for example dicaprylyl ether (Cetiol® OE), ring-opening products of epoxidized fatty acid esters with polyols, silicone oils (cyclomethicones, silicon methicone types, inter alia) and/or aliphatic or naphthenic hydrocarbons, for example squalane, squalene or dialkylcyclohexanes.

Suitable emulsifiers are, for example, nonionogenic surfactants from at least one of the following groups:
- addition products of from 2 to 30 mol of ethylene oxide and/or 0 to 5 mol of propylene oxide onto linear fatty alcohols having 8 to 22 carbon atoms, onto fatty acids having 12 to 22 carbon atoms, onto alkylphenols having 8 to 15 carbon atoms in the alkyl group, and onto alkylamines having 8 to 22 carbon atoms in the alkyl radical;
- alkyl and/or alkenyl oligoglycosides having 8 to 22 carbon atoms in the alk(en)yl radical and the ethoxylated analogs thereof;
- addition products of from 1 to 15 mol of ethylene oxide onto castor oil and/or hydrogenated castor oil;
- addition products of from 15 to 60 mol of ethylene oxide onto castor oil and/or hydrogenated castor oil;
- partial esters of glycerol and/or sorbitan with unsaturated, linear or saturated, branched fatty acids having 12 to 22 carbon atoms and/or hydroxycarboxylic acids having 3 to 18 carbon atoms, and the adducts thereof with 1 to 30 mol of ethylene oxide;
- partial esters of polyglycerol (average degree of self-condensation 2 to 8), polyethylene glycol (molecular weight 400 to 5 000), trimethylolpropane, pentaerythritol, sugar alcohols (e.g. sorbitol), alkyl glucosides (e.g. methyl glucoside, butyl glucoside, lauryl glucoside), and polyglucosides (e.g. cellulose) with saturated and/or unsaturated, linear or branched fatty acids having 12 to 22 carbon atoms and/or hydroxycarboxylic acids having 3 to 18 carbon atoms, and the adducts thereof with 1 to 30 mol of ethylene oxide;
- mixed esters of pentaerythritol, fatty acids, citric acid and fatty alcohols and/or mixed esters of fatty acids having 6 to 22 carbon atoms, methylglucose and polyols, preferably glycerol or polyglycerol,
- mono-, di- and trialkyl phosphates, and mono-, di- and/or tri-PEG alkyl phosphates and salts thereof;
- wool wax alcohols;
- polysiloxane-polyalkyl-polyether copolymers and corresponding derivatives;
- block copolymers, e.g. polyethylene glycol-30 dipolyhydroxystearates;
- polymer emulsifiers, e.g. Pemulen® grades (TR-1, TR-2) from Goodrich;
- polyalkylene glycols and
- glycerol carbonate.

The addition products of ethylene oxide and/or of propylene oxide onto fatty alcohols, fatty acids, alkylphenols or onto castor oil are known, commercially available products. These are homologous mixtures whose average degree of alkoxylation corresponds to the ratio of the amounts of ethylene oxide and/or propylene oxide and substrate with which the addition reaction is carried out. C_{12/18}-fatty acid mono- and diesters of addition products of ethylene oxide onto glycerol are known as refatting agents for cosmetic preparations.

Alkyl and/or alkenyl oligoglycosides, their preparation and their use are known from the prior art. They can be prepared by reacting glucose or oligosaccharides with primary alcohols having 8 to 18 carbon atoms. With regard to the glycoside radical, both monoglycosides, in which a cyclic sugar radical is glycosidically bonded to the fatty alcohol, and also oligomeric glycosides having a degree of oligomerization of up to, preferably, about 8, are suitable. The degree of oligomerization here is a statistical average value that is based on a homologous distribution customary for such technical-grade products.

Typical examples of suitable partial glycerides are hydroxy stearic acid monoglyceride, hydroxy stearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linoleic acid monoglyceride, linoleic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride, and the technical-grade mixtures thereof which may also comprise small amounts of triglyceride as a minor product of the preparation process. Likewise suitable are addition products of 1 to 30 mol, preferably 5 to 10 mol, of ethylene oxide onto said partial glycerides.

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate, and technical-grade mixtures thereof. Likewise suitable are addition products of 1 to 30 mol, preferably 5 to 10 mol, of ethylene oxide onto said sorbitan esters.

Typical examples of suitable polyglycerol esters are polyglyceryl-2 dipolyhydroxystearate (Dehymuls® PGPH), polyglycerol-3 diisostearate (Lameform® TGI), polyglyceryl-4 isostearate (Isolan® GI 34), polyglyceryl-3 oleate, diisostearoyl polyglyceryl-3 diisostearate (Isolan® PDI), polyglyceryl-3 methylglucose distearate (Tego Care® 450), polyglyceryl-3 beeswax (Cera Bellina®), polyglyceryl-4 caprate (Polyglycerol Caprate T2010/90), polyglyceryl-3 cetyl ether (Chimexane® NL), polyglyceryl-3 distearate (Cremophor® GS 32) and polyglyceryl polyricinoleate (Admul® WOL 1403), polyglyceryl dimerate isostearate, and mixtures thereof. Examples of further suitable polyol esters are the mono-, di- and triesters, optionally reacted with 1 to 30 mol of ethylene oxide, of trimethylolpropane or pentaerythritol with lauric acid, coconut fatty acid, tallow fatty acid, palmitic acid, stearic acid, oleic acid, behenic acid and the like.

Furthermore, zwitterionic surfactants can be used as emulsifiers. The term "zwitterionic surfactants" refers to those surface-active compounds that carry at least one quaternary ammonium group and at least one carboxylate and one sulfonate group in the molecule. Particularly suitable zwitterionic surfactants are the betaines, such as N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having in each case 8 to 18 carbon atoms in the alkyl or acyl group, and cocoacylaminoethylhydroxyethylcarboxymethyl glycinate. Particular preference is given to the fatty acid amide derivative known under the CTFA name *Cocamidopropyl Betaine.* Likewise suitable emulsifiers are ampholytic surfactants. The term "ampholytic surfactants" means those surface-active compounds that, apart from a C_{8/18}-alkyl or -acyl group in the molecule, contain at least one free amino group and at least one -COOH or -SO₃H group and are capable of forming internal salts. Examples of suitable ampholytic surfactants are N-alkylglycines, N-alkylpropionic acids, N-alkylaminobutyric acids, N-alkyliminodipropionic acids, N-hydroxyethyl-N-alkylamido-propylglycines, N-alkyltaurines, N-alkylsarcosines, 2-alkylaminopropionic acids and alkylaminoacetic acids having in each case about 8 to 18 carbon atoms in the alkyl group. Particularly preferred ampholytic surfactants are N-cocoalkyl aminopropionate, cocoacylaminoethyl aminopropionate and C_{12/18}-acylsarcosine. Finally, cationic surfactants are also suitable emulsifiers, those of the esterquat type, preferably methyl-quaternized difatty acid triethanolamine ester salts, being particularly preferred.

Fats and waxes that can be used are described in the following text. Typical examples of fats are glycerides, i.e. solid or liquid vegetable or animal products which consist essentially of mixed glycerol esters of higher fatty acids, suitable waxes are inter alia natural waxes, for example candelilla wax, carnauba wax, japan wax, esparto grass wax, cork wax, guaruma wax, rice germ oil wax, sugarcane wax, ouricury wax, montan wax, beeswax, shellac wax, spermaceti, lanolin (wool wax), uropygial grease, ceresin, ozokerite (earth wax), petrolatum, paraffin waxes, microcrystalline waxes; chemically modified waxes (hard waxes), for example montan ester waxes, sasol waxes, hydrogenated jojoba waxes, and synthetic waxes, for example polyalkylene waxes and polyethylene glycol waxes. In addition to the fats, suitable additives are also fat-like substances, such as lecithins and phospholipids. The term lecithins is understood by the person skilled in the art as meaning those glycerophospholipids which form from fatty acids, glycerol, phosphoric acid and choline by esterification. Lecithins are thus frequently also [lacuna] as phosphatidylcholines (PC). Examples of natural lecithins which may be mentioned are the cephalins, which are also referred to as phosphatidic acids and represent derivatives of 1,2-diacyl-sn-glycerol-3-phosphoric acids. By contrast, phospholipids are usually understood as meaning mono- and, preferably, diesters of phosphoric acid with glycerol (glycerophosphates), which are generally considered to be fats. In addition, sphingosines and sphingolipids are also suitable.

Examples of suitable pearlescent waxes are: alkylene glycol esters, specifically ethylene glycol distearate; fatty acid alkanolamides, specifically coconut fatty acid diethanolamide; partial glycerides, specifically stearic acid monoglyceride; esters of polybasic, optionally hydroxy-substituted carboxylic acids with fatty alcohols having 6 to 22 carbon atoms, specifically long-chain esters of tartaric acid; fatty substances, for example fatty alcohols, fatty ketones, fatty aldehydes, fatty ethers and fatty carbonates, which have a total of at least 24 carbon atoms, specifically laurone and distearyl ether; fatty acids, such as stearic acid, hydroxystearic acid or behenic acid, ring-opening products of olefin epoxides having 12 to 22 carbon atoms with fatty alcohols having 12 to 22 carbon atoms and/or polyols having 2 to 15 carbon atoms and 2 to 10 hydroxyl groups, and mixtures thereof.

Bodying agents and thickeners that can be used are described in the following text. Suitable bodying agents are primarily fatty alcohols or hydroxy fatty alcohols having 12 to 22, and preferably 16 to 18, carbon atoms, and also partial glycerides, fatty acids or hydroxy fatty acids. Preference is given to a combination of these substances with alkyl oligoglucosides and/or fatty acid N-methylglucamides of identical chain length and/or polyglycerol poly-12-hydroxystearates. Suitable thickeners are, for example, Aerosil grades (hydrophilic silicas), polysaccharides, in particular xanthan gum, guarguar, agar agar, alginates and Tyloses, carboxymethylcellulose and hydroxyethylcellulose, and also relatively high molecular weight polyethylene glycol mono-and diesters of fatty acids, polyacrylates (e.g. Carbopols® and Pemulen grades from Goodrich; Synthalens® from Sigma; Keltrol grades from Kelco; Sepigel grades from Seppic; Salcare grades from Allied Colloids), polyacrylamides, polymers, polyvinyl alcohol and polyvinylpyrrolidone, surfactants, for example ethoxylated fatty acid glycerides, esters of fatty acids with polyols for example pentaerythritol or trimethylolpropane, fatty alcohol ethoxylates having a narrowed homolog distribution or alkyl oligoglucosides, and electrolytes such as sodium chloride and ammonium chloride.

Superfatting agents which can be used are for example lanolin and lecithin, and polyethoxylated or acylated lanolin and lecithin derivatives, polyol fatty acid esters, monoglycerides and fatty acid alkanolamides, the latter also serving as foam stabilizers.

Stabilizers which can be used are metal salts of fatty acids, for example magnesium, aluminium and/or zinc stearate or ricinoleate.

Polymers that can be used are described in the following text. Suitable cationic polymers are, for example, cationic cellulose derivatives, for example a quaternized hydroxyethylcellulose obtainable under the name Polymer JR 400® from Amerchol, cationic starch, copolymers of diallylammonium salts and acryl amides, quaternized vinylpyrrolidone-vinylimidazole polymers, for example Luviquat® (BASF), condensation products of polyglycols and amines, quaternized collagen polypeptides, for example lauryldimonium hydroxypropyl hydrolysed collagen (Lamequat®L/Grünau), quaternized wheat polypeptides, polyethyleneimine, cationic silicone polymers, for example amodimethicones, copolymers of adipic acid and dimethylaminohydroxypropyldiethylenetriamine (Cartaretins®/Sandoz), copolymers of acrylic acid with dimethyl diallylammonium chloride (Merquat® 550/Chemviron), polyaminopolyamides and cross linked water-soluble polymers thereof, cationic chitin derivatives, for example quaternized chitosan, optionally in microcrystalline dispersion, condensation products from dihaloalkyls, for example dibromobutane with bisdialkylamines, for example bis-dimethylamino-1,3-propane, cationic guar gum, for example Jaguar® CBS, Jaguar® C-17, Jaguar® C-16 from Celanese, quaternized ammonium salt polymers, for example Mirapol® A-15, Mirapol® AD-1, Mirapol® AZ-1 from Miranol.

Suitable anionic, zwitterionic, amphoteric and nonionic polymers are, for example, vinyl acetate-crotonic acid copolymers, vinylpyrrolidone-vinyl acrylate copolymers, vinyl acetate-butyl maleate-isobornyl acrylate copolymers, methyl vinyl ether-maleic anhydride copolymers and esters thereof, uncrosslinked polyacrylic acids and polyacrylic acids crosslinked with polyols, acrylamidopropyltrimethylammonium chloride-acrylate copolymers, octylacrylamide-methyl methacrylate-tert-butylaminoethyl methacrylate-2-hydroxypropyl methacrylate copolymers, polyvinylpyrrolidone, vinylpyrrolidone-vinyl acetate copolymers, vinylpyrrolidone-dimethylaminoethyl methacrylate-vinylcaprolactam terpolymers, and optionally derivatized cellulose ethers and silicones.

Suitable silicone compounds are, for example, dimethylpolysiloxanes, methylphenylpolysiloxanes, cyclic silicones, and amino-, fatty-acid-, alcohol-, polyether-, epoxy-, fluorine-, glycoside- and/or alkyl-modified silicone compounds, which can either be liquid or in resin form at room temperature. Also suitable are simethicones, which are mixtures of dimethicones having an average chain length of from 200 to 300 dimethylsiloxane units and hydrogenated silicates.

Deodorants and antimicrobial agents that can be used are described in the following text. Cosmetic deodorants counteract, mask or remove body odours. Body odours arise as a result of the effect of skin bacteria on apocrine perspiration, with the formation of degradation products which have an unpleasant odour. Accordingly, deodorants comprise active ingredients which act as antimicrobial agents, enzyme inhibitors, odour absorbers or odour masking agents. Suitable antimicrobial agents are, in principle, all substances effective against gram-positive bacteria, for example 4-hydroxybenzoic acid and its salts and esters, N-(4-chlorophenyl)-N'-(3,4-dichlorophenyl)urea, 2,4,4'-trichloro-2'-hydroxydiphenyl ether (triclosan), 4-chloro-3,5-dimethylphenol, 2,2'-methylenebis(6-bromo-4-chlorophenol), 3-methyl-4-(1-methylethyl)phenol, 2-benzyl-4-chlorophenol, 3-(4-chlorophenoxy)-1,2-propanediol, 3-iodo-2-propynyl butylcarbamate, chlorohexidine, 3,4,4'-trichlorocarbanilide (TTC), antibacterial fragrances, thymol, thyme oil, eugenol, oil of cloves, menthol/mint oil, farnesol, phenoxyethanol, glycerol monocaprate, glycerol monocaprylate, glycerol monolaurate (GML), diglycerol monocaprate (DMC), salicylic acid N-alkylamides, for example n-octylsalicylamide or n-decylsalicylamide.

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen® CAT). The substances inhibit enzyme activity, thereby reducing the formation of odour. Other substances which are suitable esterase inhibitors are sterol sulfates or phosphates, for example lanosterol, cholesterol, campesterol, stigmasterol and sitosterol sulfate or phosphate, dicarboxylic acids and esters thereof, for example glutaric acid, monoethyl glutarate, diethyl glutarate, adipic acid, monoethyl adipate, diethyl adipate, malonic acid and diethyl malonate, hydroxycarboxylic acids and esters thereof, for example citric acid, malic acid, tartaric acid or diethyl tartrate, and zinc glycinate.

Suitable odour absorbers are substances which are able to absorb and largely retain odour-forming compounds. They lower the partial pressure of the individual components, thus also reducing their rate of diffusion. It is important that in this process perfumes must remain unimpaired. Odour absorbers are not effective against bacteria. They comprise, for example, as main constituent, a complex zinc salt of ricinoleic acid or specific, largely odour-neutral fragrances which are known to the person skilled in the art as "fixatives", for example extracts of labdanum or styrax or certain abietic acid derivatives. The odour masking agents are fragrances or perfume oils, which, in addition to their function as odour masking agents, give the deodorants their respective fragrance note. Perfume oils which may be mentioned are, for example, mixtures of natural and synthetic fragrances. Natural fragrances are extracts from flowers, stems and leaves, fruits, fruit peels, roots, woods, herbs and grasses, needles and branches, and resins and balsams. Also suitable are animal raw materials, for example civet and castoreum. Typical synthetic fragrance compounds are products of the ester, ether, aldehyde, ketone, alcohol and hydrocarbon type. Fragrance compounds of the ester type are, for example, benzyl acetate, p-tert-butylcyclohexyl acetate, linalyl acetate, phenylethyl acetate, linalyl benzoate, benzyl formate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cyclamen aldehyde, hydroxycitronellal, lilial and bourgeonal, the ketones include, for example, the ionones and methyl cedryl ketone, the alcohols include anethole, citronellol, eugenol, isoeugenol, geraniol, linalool, phenylethyl alcohol and terpineol, and the hydrocarbons include mainly the terpenes and balsams. Preference is, however, given to using mixtures of different fragrances which together produce a pleasing fragrance note. Ethereal oils of relatively low volatility, which are mostly used as aroma components, are also suitable as perfume oils, e.g. sage oil, camomile oil, oil of cloves, melissa oil, mint oil, cinnamon leaf oil, linden flower oil, juniper berry oil, vetiver oil, olibanum oil, galbanum oil, labdanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Antiperspirants reduce the formation of perspiration by influencing the activity of the eccrine sweat glands, thus counteracting underarm wetness and body odor. Aqueous or anhydrous formulations of antiperspirants typically comprise one or more of the following ingredients: astringent active ingredients, oil components, nonionic emulsifiers, coemulsifiers, bodying agents, auxiliaries, for example thickeners or complexing agents, and/or nonaqueous solvents, for example ethanol, propylene glycol and/or glycerol.

Suitable astringent antiperspirant active ingredients are primarily salts of aluminum, zirconium or of zinc. Such suitable antihydrotic active ingredients are, for example, aluminum chloride, aluminum chlorohydrate, aluminum dichlorohydrate, aluminum sesquichlorohydrate and complex compounds thereof, e.g. with 1,2-propylene glycol, aluminum hydroxyallantoinate, aluminum chloride tartrate, aluminum zirconium trichlorohydrate, aluminum zirconium tetrachlorohydrate, aluminum zirconium penta-chlorohydrate and complex compounds thereof, e.g. with amino acids, such as glycine. In addition, customary oil-soluble and water-soluble auxiliaries may be present in antiperspirants in relatively small amounts. Such oil-soluble auxiliaries may, for example, be anti-inflammatory, skin-protective or perfumed ethereal oils, synthetic skin-protective active ingredients and/or oil-soluble perfume oils.

Customary water-soluble additives are, for example, preservatives, water-soluble fragrances, pH regulators, e.g. buffer mixtures, water-soluble thickeners, e.g. water-soluble natural or synthetic polymers, for example xanthan gum, hydroxyethylcellulose, polyvinylpyrrolidone or high molecular weight polyethylene oxides.

Film formers that can be used are described in the following text. Customary film formers are, for example, chitosan, microcrystalline chitosan, quaternized chitosan, polyvinylpyrrolidone, vinylpyrrolidone-vinyl acetate copolymers, polymers of the acrylic acid series, quaternary cellulose derivatives, collagen, hyaluronic acid and salts thereof, and similar compounds.

Suitable antidandruff active ingredients are piroctone olamine (1-hydroxy-4-methyl-6-(2,4,4-trimethylpentyl)-2-(1H)-pyridinone monoethanolamine salt), Baypival® (climbazole), Ketoconazole®, (4-acetyl-1-{-4-[2-(2,4-dichlorophenyl) r-2-(1H-imidazol-1-ylmethyl)-1,3-dioxylan-c-4-ylmethoxyphenyl}piperazine, ketoconazole, elubiol, selenium disulfide, colloidal sulfur, sulfur polyethylene glycol sorbitan monooleate, sulfur ricinol polyethoxylate, sulfur tar distillates, salicyclic acid (or in combination with hexachlorophene), undecylenic acid monoethanolamide sulfosuccinate Na salt, Lamepon® UD (protein undecylenic acid condensate), zinc pyrithione, aluminum pyrithione and magnesium pyrithione/dipyrithione magnesium sulfate.

The swelling agents for aqueous phases may be montmorillonites, clay mineral substances, Pemulen, and alkyl-modified Carbopol grades (Goodrich).

Suitable insect repellents are N,N-diethyl-m-toluamide, 1,2-pentanediol or ethyl butylacetylaminopropionate.

To improve the flow behavior, hydrotropes, for example ethanol, isopropyl alcohol, or polyols, can be used. Polyols which are suitable here preferably have 2 to 15 carbon atoms and at least two hydroxyl groups. The polyols can also contain further functional groups, in particular amino groups, or be modified with nitrogen. Typical examples are:
- glycerol;
- alkylene glycols, for example, ethylene glycol, diethylene glycol, propylene glycol, butylene glycol, hexylene glycol, and polyethylene glycols with an average molecular weight of from 100 to 1 000 daltons;
- technical-grade oligoglycerol mixtures with a degree of self-condensation of from 1.5 to 10, for example, technical-grade diglycerol mixtures with a diglycerol content of from 40 to 50% by weight;
- methylol compounds, such as trimethylolethane, trimethylolpropane, trimethylolbutane, pentaerythritol and dipentaerythritol;
- lower alkyl glucosides, in particular those with 1 to 8 carbon atoms in the alkyl radical, for example methyl and butyl glucoside;
- sugar alcohols with 5 to 12 carbon atoms, for example sorbitol or mannitol,
- sugars with 5 to 12 carbon atoms, for example glucose or sucrose;
- amino sugars, for example glucamine;
- dialcohol amines, such as diethanolamine or 2-amino-1,3-propanediol.

Suitable preservatives are, for example, phenoxyethanol, formaldehyde solution, parabenes, pentanediol or sorbic acid, and the other classes of substance listed in Annex 6, Part A and B of the Cosmetics Directive.

Perfume oils which may be used are preferably mixtures of natural and synthetic fragrances. Natural fragrances are extracts from flowers (lily, lavender, rose, jasmine, neroli, ylang-ylang), stems and leaves (geranium, patchouli, petitgrain), fruits (aniseed, coriander, cumin, juniper), fruit peels (bergamot, lemon, orange), roots (mace, angelica, celery, cardamom, costus, iris, calmus), woods (pine wood, sandalwood, guaiac wood, cedarwood, rosewood), herbs and grasses (tarragon, lemon grass, sage, thyme), needles and branches (spruce, fir, pine, dwarf-pine), resins and balsams (galbanum, elemi, benzoin, myrrh, olibanum, opoponax). Also suitable are animal raw materials, for example civet and castoreum. Typical synthetic fragrance compounds are products of the ester, ether, aldehyde, ketone, alcohol and hydrocarbon type. Fragrance compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethylmethylphenyl glycinate, allyl cyclohexylpropionate, styrallyl propionate and benzyl salicylate. The ethers include, for example, benzyl ethyl ether, the aldehydes include; for example, the linear alkanals having 8 to 18 carbon atoms, citral, citronellal, citronellyloxyacetaldehyde, cyclamen aldehyde, hydroxycitronellal, lilial and bourgeonal, and the ketones include, for example, the ionones, α-isomethylionone and methyl cedryl ketone, the alcohols include anethole, citronellol, eugenol, isoeugenol, geraniol, linalool, phenylethyl alcohol and terpineol, and the hydrocarbons include predominantly the terpenes and balsams. Preference is, however, given to using mixtures of different fragrances which together produce a pleasing fragrance note. Ethereal oils of relatively low volatility, which are mostly used as aroma components, are also suitable as perfume oils, e.g. sage oil, camomile oil, oil of cloves, melissa oil, mint oil, cinnamon leaf oil, linden blossom oil, juniper berry oil, vetiver oil, olibanum oil, galbanum oil, labolanum oil and lavandin oil. Preference is given to using bergamot oil, dihydromyrcenol, lilial, lyral, citronellol, phenylethyl alcohol, α-hexylcinnamaldehyde, geraniol, benzylacetone, cyclamen aldehyde, linalool, boisambrene forte, ambroxan, indole, hedione, sandelice, lemon oil, mandarin oil, orange oil, allyl amyl glycolate, cyclovertal, lavandin oil, clary sage oil, β-damascone, geranium oil bourbon, cyclohexyl salicylate, Vertofix coeur, iso-E-super, Fixolide NP, evernyl, iraldein gamma, phenylacetic acid, geranyl acetate, benzyl acetate, rose oxide, romilat, irotyl and floramat alone or in mixtures.

Dyes which can be used are the substances which are approved and suitable for cosmetic purposes. These dyes are normally used in concentrations of from 0.001 to 0.1 % by weight, based on the total mixture.

### EXAMPLES

### Example 1: preparation of sinapinic acid (by the Knoevenagel condensation route)

In a 1 liter reaction vessel with stirrer, reflux condenser and thermometer 73.4 g (0.4 mol) of syringaldehyde, 95.6 g (0.92 mol) malonic acid, 220 g pyridine and 6 g piperidine were introduced and vigorously stirred. The mixture was heated and refluxed for 7 hours at temperatures between 85 and 89 °C. Upon cooling to room temperature, the reaction mass was neutralized using 300 g concentrated hydrochloric acid. A precipitate was formed by standing over night, which was collected, filtrated, washed with water and dried at 70 °C under vacuum. Yields were 39.2 g of sinapinic acid raw material. Identification of the reaction product as sinapinic acid was done by IR and NMR measurements.

### Example 2: recrystallization of sinapinic acid

23.3 g of sinapinic acid raw material were dissolved in 100 ml 80% ethanol by heating the solution under stirring. Upon slowly cooling to room temperature, cristallisation occurred yielding 11,9 g of red brown crystals. The mother liquor was concentrated to yield another 2.3 g crystallized material. A second crystallization step was carried out to produce nearly egg-coloured crystals, melting point 201 - 202 °C, as reported in the literature.

### Example 3: preparation of sinapinic acid by the Perkin route

25 g (0.11 mol) 4-acetoxy-3,5-dimethoxybenzaldehyde, 17.3 g (0.17 mol) acetanhydride and 4.9 g sodium acetate were mixed in a 0.5 liter reaction vessel with stirrer, reflux condenser and thermometer and heated to 145 -150°C for 8 hours. After cooling 17.3 g acetanhydride and 4.9 g sodium acetate were again added and heated for another 4 hours.

After cooling the reaction mass was dissolved in 1 liter water and 75 ml concentrated sodium hydroxide were added.

The solution is then adjusted to pH 3 by addition of 125 g concentrated hydrochloric acid. Upon this precipitation occurs , which is filtrated, dried under vacuum at 70°C. IR and NMR spectroscopy of an intensive orange coloured powder show the expected bands and signals of sinapinic acid.

### Example 4: hydrogenation of sinapinic acid

15.8 g (0.09 mol) sinapinic acid were dissolved in 180 ml ethanol and introduced in an autoclave reactor. Under the appropriate security precautions 2 g hydrogenation catalyst 5% palladium on charcoal were added. The autoclave was closed, washed with nitrogen and charged with 100 atm hydrogen. The mixture was heated at 120°C for 5 hours. After cooling and degassing, the solution was filtrated to get rid off the catalyst and concentrated to yield 15.2 g dihydrosinapinic acid after drying. IR and NMR spectra were consistent with the expected bands and signals.

This dihydrosinapinic product thus obtained was used in the following tests.

### Example 6 - Cytophotoprotection of human fibroblasts against UVA radiation

The cytoprotection against UVA irradiation has been evaluated by a test on human fibroblasts because UVA radiation penetrates through the epidermis until the dermis where it induces oxidative stress, mainly by activation of photosensitising biological components, which catalyse the formation of ROS like anion superoxide, hydrogen peroxide and singlet oxygen, and lipoperoxydation of the cell membrane. These oxidative stress effects are evaluated in vitro due to measuring of the level of released malondialdehyde (MDA) (Morlière P., Moisan A., Santus R., Huppe G., Mazière J.C., Dubertret L. UV-A induced lipid peroxydation in cultured human fibroblasts. Biochim. Biophys. Acta (1991) Vol 1084, pp 261-269). The lipoperoxides formed after UVA irradiation undergo a decay into malondialdehyde which can form cross-links between many biological molecules like proteins with inhibition of enzymes and nucleic bases with risk of mutagenesis.

Human fibroblasts were inoculated with growth medium (DMEM+FCS) and incubated 3 days at 37°C, with 5% CO₂. The growth medium was then exchanged by a medium containing the ingredient to be tested and was incubated 2 days at 37°C under an atmosphrere with 5 % of carbon dioxide. After the exchange of the medium bya balanced salt solution, the cell culture was irradiated with UVA radiation (20J/cm²). Cell proteins were measured, and MDA released in the supernatant was determined spectrophotometrically.

**Table 2**

| Results in % against control (average of 2 assays, each carried out three times): | | | |
|---|---|---|---|
| Product | Dose (% w/v) | Cell proteins | MDA released |
| Control (not irradiated) | - | 100 | 0 |
| Control / UVA (20 J/cm2) | - | 120 | 100 |
| Dihydrosinapinic acid | 0.01 | 121 | 47 |
| | 0.03 | 140 | 12 |

The UVA irradiation has induced a release of MDA. After incubation of the fibroblasts with dihydrosinapinic acid, a strong protection of the cells against UVA-induced MDA released was obtained.

### Example 7. Anti-inflammatory activity on human leucocytes cultured in vitro.

Human leukocytes (Polymorphonuclear neutrophilic granulocytes) were incubated with dihydrosinapinic acid during 1 day at 37°C under an atmosphere with 5 % carbon dioxide. The respiratory burst was induced by addition of a yeast extract ("Zymosan"). After incubation for 0.5 hours at 37 °C under an atmosphere with 5 % carbon dioxide the cell number was measured using an automatic cell counter and the rate of released reactive oxygen species was recorded by a luminescent reaction with luminol. (Pieper G.M., Gross G.J. EMD 52692 (bimakalim) a new potassium channel opener, attenuates luminol-enhanced chemiluminescence and superoxide anion radical formation by Zymosan-activated polymorphonuclear leukocytes (**Immunopharmacology (1992) vol 23, pp 191-197**).

**Table 3**

| Results in % against control (average of 2-3 assays, each carried out 3 times): | | | |
|---|---|---|---|
| Product | Dose (% w/v) | Cell number | Released ROS |
| Control | - | 100 | 100 |
| Dihydrosinapinic acid | 0.0001 | 99 | 25 |
| | 0.0003 | 98 | 13 |

The Zymosan has activated the polymorphonuclear neutrophilic granulocytes resulting in the release of reactive oxygen species. Dihydrosinapinic acid has inhibited this respiratory burst.

### Example 8. Anti-free radical activity

Free radicals (FR) are reactive chemical species, characterised by at least one unpaired electron. FR can originate from oxygen by spontaneous biological mechanisms such as the respiratory chain in mitochondria, or by natural biological processes such as inflammation. Oxidative stress like UV or chemical pollutants also induce the rise of the level of free radicals which provokes damages on all cellular and tissue constituents (lipids, proteins, sugars and nucleic bases) of living organisms. the FR toxicity is enhanced by the oxygen level and constitutes a key process in ageing, in the appearance of diseases such as cancer, diabetes etc.

The anti-free radical (anti-FR) activity has been evaluated by chemical tests for the evaluation of the potential to scavenge free radicals R° (DPPH° test) and hydroxyl radicals HO°. Furthermore the anti-FR activity has been evaluated by biochemical tests to address the potential for scavenging of a kind of reactive oxygen species (ROS), so called superoxide anions (02°). The 02° originates mainly from lipoxygenase activity, displayed by leukocytes along the leukotriens synthesis during inflammatory process (Bouclier M & Hensby CN. Prostaglandines et leucotriènes en physiologie cutanée. Bulletin d'Esthétique Dermatologique et de Cosmétologie, (1986) pp 17-22).

### Anti free radicals : DPPH° (diphenylpicryl-hydrazyl) test

The DPPH° scavenging effect of dihydrosinapinic acid has been measured by recording the optical density at 513 nm (DEBY C. Relation entre les acides gras essentiels et le taux des antioxydants tissulaires chez la souris. SOCIETE BELGE DE BIOLOGIE, séance du 19 décembre 1970, pp: 2675-2680).
The IC₅₀ (Inhibitory Concentration 50 %: dose for which 50 % of DPPH° is scavenged, average of two assays) of dihydrosinapinic acid is 0.0006 % (w/v).

### Anti hydroxyl radicals : HO° test with deoxyribose (Fenton's reaction)

The hydroxyle radical HO* (formed by H₂O₂ in presence of Fe++) oxidises deoxyribose, then a pink compound is formed by condensation of thiobarbituric acid with the oxidised form of deoxyribose. The level of oxidised deoxyribose is determined by measurement of the optical density at 532 nm. As no EDTA was used in the assays, dihydrosinapinic acid may act by scavenging hydroxyle radicals, or by forming an inactive complex with ferrous ions (Halliwell B, Gutteridge JMC, Aruoma Ol. The deoxyribose method: a simple "Test-Tube" assay for determination of rate constants for reactions of hydroxyl radicals. Analytical Biochemistry (1987) Vol 165, pp 215-9)
The IC₅₀ (average of 2 assays) of dihydrosinapinic acid is 0.025 % (w/v).

### Inhibition test on lipoxygenase

Lipoxygenase was incubated with a specific substrate (unsaturated fatty acid) and the ingredient to be tested. Then the rate of released superoxide anions was evaluated by Luminol luminescent probes.
The IC₅₀ (mean of 2 assays) of dihydrosinapinic acid was 0.0002 % (w/v).

## Claims

1. The use of a compound having formula I 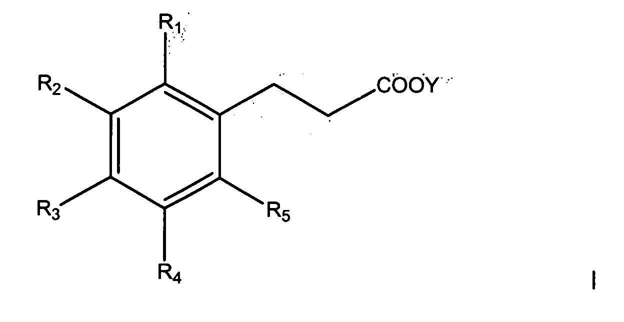 wherein
Y is selected from the group consisting of H, an alkyl group, a phenyl group, Na, K or NH₄,
R₁, R₂, R₃, R₄ and R₅ are independently selected from the group consisting of H and -O-R₆, wherein R₆ is selected from the group consisting of H and a linear or branched, saturated or unsaturated alkyl group comprising 1 to 6 carbon atoms,
and wherein at least one of the substituents R₁, R₂, R₃, R₄ and R₅ is not H,
for the manufacture of a composition for the cosmetic treatment of human skin, scalp or hair.

2. The use of a compound having formula I 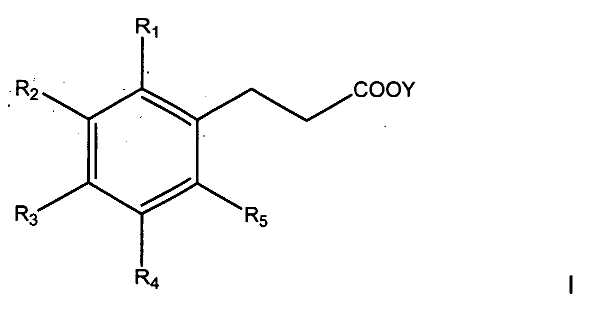 wherein
Y is selected from the group consisting of H, an alkyl group, a phenyl group, Na, K or NH₄,
R₁, R₂, R₃, R₄ and R₅ are independently selected from the group consisting of H and -O-R₆, wherein R₆ is selected from the group consisting of H and a linear or branched, saturated or unsaturated alkyl group comprising 1 to 6 carbon atoms,
and wherein at least one of the substituents R₁, R₂, R₃, R₄ and R₅ is not H,
for the cosmetic treatment of human skin, scalp or hair.

3. The use according to claim 1 or 2, wherein
Y is selected from the group consisting of H, methyl, ethyl and propyl,
R₁ = R₂ = H,
R₃, R₄ and R₅ are independently selected from the group consisting of H, -OH and -O-methyl,
and wherein at least one of the substituents R₃, R₄ and R₅ is not H.

4. The use according to claim 1 or 2, wherein the compound is dihydrosinapinic acid.

5. The use according to claim 1, wherein the composition comprises
a) the compound having formula I and
b) at least one cosmetic adjuvant.

6. The use according to claim 2, wherein the compound is used in a composition comprising
a) the compound having formula I and
b) at least one cosmetic adjuvant.

7. The use according to claim 6 or 7, wherein the cosmetic adjuvant is selected from the group consisting of oily bodies, surfactants, emulsifiers, fats, waxes, pearlescent waxes, bodying agents, thickeners, superfatting agents, stabilizers, polymers, silicone compounds, lecithins, phospholipids, biogenic active ingredients, deodorants, antimicrobial agents, antiperspirants, film formers, antidandruff agents, swelling agents, insect repellents, hydrotropes, solubilizers, preservatives, perfume oils and dyes.

8. The use according to any of claims 1 to 7 wherein the cosmetic treatment comprises the bringing about of
an anti-ageing or anti-photoageing effect (related to UV irradiation or IR irradiation)
or a protecting effect against UV-A-radiation ("UV-A-cytophotoprotection")
or an anti-inflammatory, or an anti-irritant effect, or an anti-itching effect or an appeasing effect
or an anti-protease effect
or an anti-lipoxygenase effect.
